# EUROPEAN PATENT SPECIFICATION

(11) **EP 1 589 340 B1**
(45) Date of publication and mention of the grant of the patent: **06.01.2010**
(21) Application number: 04700535.0
(22) Date of filing: 07.01.2004
(51) Int. Cl.: G01N 33/53, G01N 33/543, G01N 37/00, G01G 3/13

(54) **REACTIVE CHIP AND METHOD FOR DETECTING BOND OF TARGET SUBSTANCE USING THAT CHIP**
REAKTIVER CHIP UND VERFAHREN ZUM ERKENNEN DER BONDIERUNG EINER ZIELSUBSTANZ UNTER VERWENDUNG DIESES CHIPS
PUCE REACTIVE ET PROCEDE POUR DETECTER LA LIAISON D'UNE SUBSTANCE CIBLE AU MOYEN DE CETTE PUCE

(30) Priority: 07.01.2003 JP 2003001577
(43) Date of publication of application: 26.10.2005
(73) Proprietor: NGK INSULATORS, LTD., Nagoya-City, Aichi Pref. 467-8530 (JP)
(72) Inventor: Yoshida, Yasuko, Nagoya-shi, Aichi 454-0943 (JP); Hirota, Toshikazu, Nagoya-shi, Aichi 458-0014 (JP); Takeuchi, Yukihisa, Nishikamo-gun, Aichi 470-0204 (JP)
(74) Representative: Paget, Hugh Charles Edward
(86) International application number: PCT/JP2004/000038
(87) International publication number: WO 2004/061451

(56) References cited:
- EP-A- 0 215 669
- EP-A- 0 984 252
- WO-A-93/22678
- WO-A2-01/36681
- JP-A- 7 075 598
- JP-A- 7 508 831
- JP-A- 2000 321 117
- JP-A- 2002 048 797
- JP-A- 2003 307 481

## Description

### Technical Field

The present invention relates to a reactive chip and to a method of detecting a target substance using the chip. More particularly, the invention relates to a novel reactive chip which eliminates any mishybridization between the target substance and a capture probe, allows an accurate detection to be accomplished within a short time period and gives a wide range of the detection means and the detection targets to be selected, as well as a novel method for detecting a target substance utilizing such a chip.

### Background Art

For the purpose of a large scale rapid analysis of a gene structure or a gene expression mode, various reactive chips are employed. Such a reactive chip is formed by arranging and fixing several hundreds to several ten hundreds or more of different capture probes as spots on a support such as a glass slide, and enables the identification or quantification of a target substance in a sample using as an index the presence or absence of the binding of the target substance labeled for example with a fluorescent substance to the capture probes. The capture probe fixed on the chip varies depending on the type of the target substance to be analyzed. For example, when a DNA or RNA is the target substance, then a capture probe employed is one capable of forming a complementary bond (hybridization) with them, such as a double-stranded or single-stranded DNA fragment or polynuclotide chain, oligonicleotide chan, whereby forming a so-called DNA chip (or DNA array) (see for example Patent Document 1-4, Non-Patent Documents 1 and 2). On the other hand, in a protein chip, a protein or peptide and a receptor or antibody which reacts with it specifically are the constituents of the target substance-capture probe relationship.

The binding of a target substance in a reactive chip is verified for example by a procedure comprising bringing a sample containing a labeled target substance (sample solution) into contact with the reactive chip, and allowed to react for a certain time period to bind the target substance to a capture probe, followed by removing any non-binding substance, followed by detecting the position of the label on the reactive chip to reveal which capture probe is bound to the target substance. Also by measuring the signal intensity of the label, the target substance can be quantified.

A conventional reactive chip employs a prolonged period for the reaction between a sample solution and a the reactive chip for the purpose of allowing a target substance in the sample solution to get closer and bind to the respective capture probe as being driven by a spontaneous diffusion. As a result, it takes a problematically long time to obtain the results of the detection.

The accuracy of the detection of a target substance by a reactive chip depends on the specific binding between a target substance and a capture probe. In the case for example of a DNA chip, it is ideal that a target DNA and a probe DNA bind in a complete complementarity with each other, but in fact the target DNA may bind to the probe DNA even in the presence of several mismatches. Especially when the target DNA is driven by a spontaneous diffusion in the sample solution to get closer to the probe DNA, the risk of such a mismatch binding (mishybridization) becomes extremely high.

As a DNA chip capable of overcoming the problems mentioned above, an invention of the Patent Document 5 is known (nano-chip). This nano-chip has a probe DNA fixed on the surface of an electrode, to which a positive charge is applied to the electrode while allowing a target DNA to hybridize with a probe DNA. Since a DNA fragment (target DNA) is charged negatively, it can get closer and bind to the positively charged probe DNA within a short time period. Then after the completion of the hybridization reaction, a negative charge is applied to the electrode. As a result, the probe DNA and the target DNA are both charged negatively, and the target DNA undergoing a mismatch binding to the probe DNA is repelled by the probe DNA, whereby allowing the target DNA binding in a correct complementarity to be remain exclusively together with the probe DNA.

Nevertheless, this nano-chip can not be applied to a protein chip utilizing a protein or peptide since it utilizes the nature of a DNA usually carrying a negative charge.

EP-A-215669 discloses an analytical device in which receptor materials, for target capture, are immobilized on the surface of piezoelectric crystals. Weight changes caused by capture of targets cause change of resonant frequencies, which are detected. The type of target captured can be determined.

EP-A-984252 discloses a structure having in combination the features of the first part of present claim 1.

### References

Patent Document 1: USP No.5,474,796.
Patent Document 2: USP No.5,605,662.
Patent Document 3: WO95/25116
Patent Document 4: WO95/35505
Patent Document 5: JP-W-2001-501301
Non-Patent Document 1: Schena, M. et al., Proc. Natl. Acad. Sci. USA. 93:10614-10619, 1996
Non-Patent Document 2: Heller, R. A. et al., Proc. Natl. Acad. Sci. USA. 94:2150-2155, 1997

The present invention has been established based on the circumstance described above, and its objective is to provide a novel reactive chip capable of reducing the reaction period, applying a wide-ranging of target substance, preventing a mismatch binding efficiently, and enabling a highly accurate detection.

The invention provides a reactive chip as set out in claim 1.

Preferably the lead wire for each of the first and second electrodes is independent from the respective lead wire of the electrodes of each other vibration-generating part.

Preferably the lead wire for one of the first and second electrodes is in common, for said vibration-generating parts.

There may be means for measuring a resonance frequency of the piezoelectric/electrostrictive element.

Preferably the surface of the first electrode is a capture probe-fixing surface and the first electrode and the second electrode are connected not only with an alternating-current power source but also with a direct-current power source.

Preferably the kind of capture probes fixed on a vibration area is different from the capture probes of other vibration areas.

Preferably the chip employs an arrangement of three or more vibration areas in a line or four or more vibration areas in a matrix of n × m wherein n is 2 or more and m is 2 or more, with identical capture probes being fixed in each vibration area in identical lines.

Alternatively, the chip employs an arrangement of three or more vibration areas in a line or four or more vibration areas in a matrix of n × m wherein n is 2 or more and m is 2 or more, with a capture probe which binds to a different site of a target substance being fixed in each vibration area in an identical line.

The invention also provides a method for detecting a target substance using the reactive chip of the invention. Preferably the method comprises bringing a labeled target substance-containing sample into contact with the capture probes on the reactive chip while vibrating the vibration area of the reactive chip, followed by terminating the vibration of the vibration area and detecting the target substance bound to the capture probe using the label as an index.

### Brief Description of the Drawings

Fig. 1 shows a lateral view of one example of a basic structure of an inventive reactive chip.
Fig. 2 shows a lateral view of another example of a basic structure of an inventive reactive chip.
Fig. 3 shows a plain and lateral view of still another example of a basic structure of an inventive reactive chip.
Fig. 4 shows a lateral view of one example of an inventive reactive chip.
Fig. 5 shows a lateral view of another example of an inventive reactive chip.
Fig. 6 shows a lateral view of still another example of an inventive reactive chip.
Fig. 7 shows a plain and lateral view of a further example of an inventive reactive chip.
Fig. 8 shows a plain view of a still further example of an inventive reactive chip.
Fig. 9 is a schematic view of an example of the arrangement of capture probes in an inventive reactive chip. A square shows a vibration area, and different alphabetical symbols represent different capture probes.
Fig. 10 is a schematic view of another example of the arrangement of capture probes in an inventive reactive chip. A square shows a vibration area, and different alphabetical symbols represent different capture probes.
Fig. 11 shows a schematic view of still another example of the arrangement of capture probes in an inventive reactive chip (left) and a schematic view exemplifying the binding condition of a normal chromosome when this reactive chip is employed to detect a chromosomal aberration and the like (right). A square shows a vibration area, and a wiggle line communicating the vibration areas arranged laterally shows a vibrator. Different alphabetical symbols and different numbers represent different capture probes.
Fig. 12 is a schematic view of still another example of the arrangement of capture probes in an inventive reactive chip. A square shows a vibration area, and different alphabetical symbols and different numbers represent different capture probes.
Fig. 13 shows an example of the arrangement of capture probes exemplified in Fig. 11 (left) and a schematic view exemplifying the binding condition observed when this reactive chip is employed to detect a chromosomal aberration (amplification) (right). A square shows a vibration area, and a wiggle line communicating the vibration areas arranged laterally shows a vibrator. Different alphabetical symbols and different numbers represent different capture probes.
Fig. 14 shows an example of the arrangement of capture probes exemplified in Fig. 11 (left) and a schematic view exemplifying the binding condition observed when this reactive chip is employed to detect a chromosomal aberration (deletion) (right). A square shows a vibration area, and a wiggle line communicating the vibration areas arranged laterally shows a vibrator. Different alphabetical symbols and different numbers represent different capture probes.
Fig. 15 shows an example of the arrangement of capture probes exemplified in Fig. 12 (left) and a schematic view exemplifying the binding condition observed when this reactive chip is employed to detect a chromosomal aberration (insertion) (right). A square shows a vibration area, and different alphabetical symbols and different numbers represent different capture probes.
Fig. 16 shows an example of the arrangement of capture probes exemplified in Fig. 12 (left) and a schematic view exemplifying the binding condition observed when this reactive chip is employed to detect a chromosomal aberration (substitution) (right). A square shows a vibration area, and different alphabetical symbols and different numbers represent different capture probes.

### Legend

- 11: First electrode
- 12: Second electrode
- 13,14: Lead wire
- 20: Piezoelectric/electrostrictive element
- 30: Support
- 31: Thin area
- 32: Thick area
- 40: Vibration-generating part
- 50: Vibration area
- 60: Capture probe
- 70: Coating layer

### Best Mode for Carrying Out the Invention

The first invention in this application is a reactive chip comprising capture probes fixed on each of three or more vibration areas arranged on a support, the capture probes being able to binding to a target substance.

A "support" is a glass slide, ceramic plate, resin plate such as a plastic plate, metal plate and the like which are employed in ordinary DNA chips and protein chips. A "capture probe" is a biological molecule which binds specifically to a target substance. For example, when the target substance is a DNA fragment (such as a cDNA) derived from a genome DNA, then the capture probe employed is one capable of hybridizing with such a DNA fragment on the basis of the complementarity as a single-stranded DNA fragment, RNA fragment, nucleotide chain (polynucleotide of 100 bases or more or oligonicleotide of less than 100 bases) and the like. When the target substance is a protein, the capture probe employed may be a protein (for example a receptor protein) or peptide which binds specifically to a part of the amino acid sequence of such a protein, or an antibody which can bind an epitope of the protein as well as its Fab, F(ab')₂, Fv fragment and the like. In addition, a carbohydrate chain-carrying composite biological molecule, biological tissue specimen, cell, yeast and other microorganism may serve as a capture probe.

Such a capture probe can be arranged on a support by a known method similarly to a conventional DNA chip or protein chip. For example in the case of a DNA chip, a DNA fragment (for example of about 25mer) is synthesized on a support, or a DNA fragment may be fixed on a support by a spotting method. When the spotting method is employed, the ink jet method disclosed in JP-A-2001-116750 and JP-A-2001-186881 is employed preferably. After the spotting step, a procedure similar to an ordinary reactive chip production, involving an addition of water (keeping the humidity at about 80% for a certain time period) into a spot, a baking at a high temperature, a fixation by a chemical treatment and the like, may be conducted to fix each spot on the support. Also in the production of a reactive chip by the spotting method, a repetitive spotting process disclosed in JP-A-2001-186880 may be conducted. When a protein, peptide, tissue specimen or cell is fixed, then a biologically specific adsorbent or organic polymer is coated preliminarily onto the fixation surface, and on this coating layer then the capture probes may be fixed.

In a reactive chip of the invention, capture probes are fixed on each of the three or more vibration areas. Each "vibration area" is arranged on a support at a distance of 100 to 1000 µm from each other, and the shape of each vibration area may be a circle whose diameter is about 50 to 500 µm or a square whose one side is about 50 to 500 µm in length. This vibration area is a layer which vibrates at a certain frequency and amplitude. Such a vibration area may be formed by providing a suitable vibration-generating device (for example, electromagnet or low frequency-generating device) on the lower surface of the probe-fixing surface of the support, with the configuration of the second invention being preferred here.

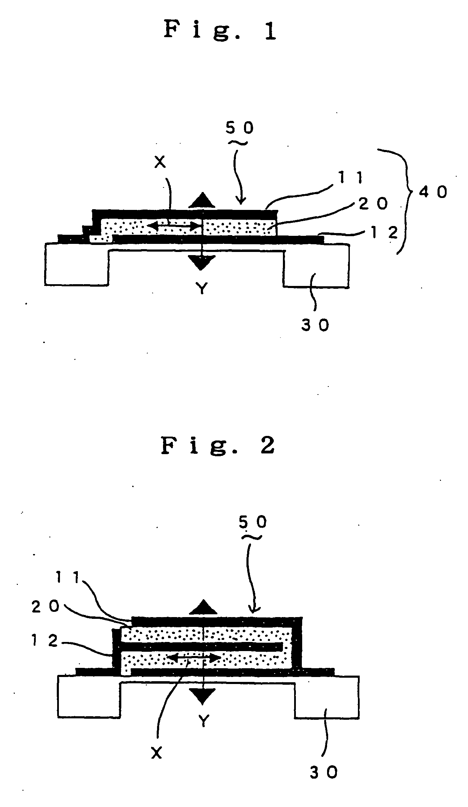

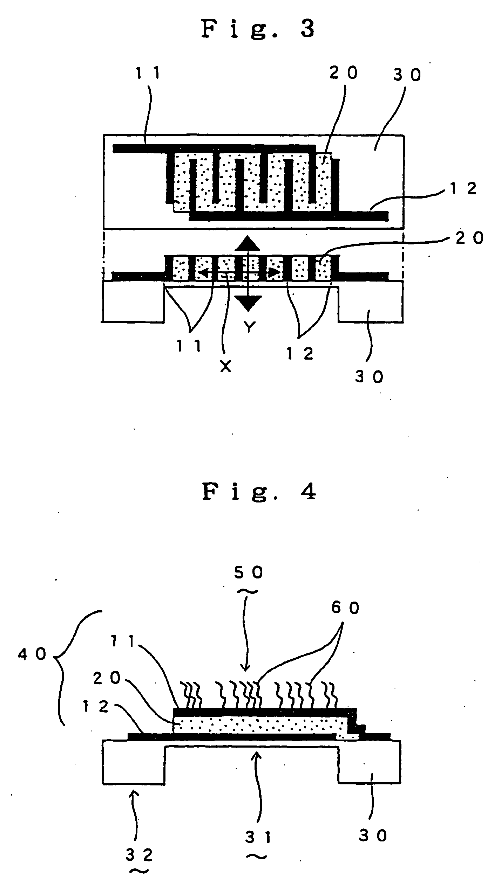

In the reactive chip, each vibration area has a vibration-generating part having a first electrode and a second electrode between which a piezoelectric/electrostrictive element is sandwiched. In this case, the vibration area may have a structure shown for example in Fig. 1. Thus, the example shown in Fig. 1 comprises the piezoelectric/electrostrictive element (20) inserted between the first electrode (11) and the second electrode (12) to form the vibration-generating part (40), which is fixed on the support (30) whereby giving the vibration area (50). When an alternating voltage is applied to the first electrode (11) and the second electrode (12), the piezoelectric/electrostrictive element (20) undergoes expansion and contraction continuously in the direction of the arrow X in response to a frequency of voltage, but the support (30) does not undergo such expansion and contraction, resulting in a vibration in the direction of the arrow Y in the vibration area (50). The cycle and the amplitude of a vibration may vary depending on a frequency and a magnitude of voltage, respectively. The first electrode (11), the second electrode (12) and the piezoelectric/electrostrictive element (20) may also be in a relationship with each other as shown in Fig. 2 or Fig. 3. In the example shown by Fig. 2, the first electrode (11) is provided on the upper and lower surfaces of the piezoelectric/electrostrictive element (20), while the second electrode (12) is inserted into the piezoelectric/electrostrictive element (20). In this configuration, an increase in the expansion and contraction of the piezoelectric/electrostrictive element (20) in the direction of X results in an increased vibration in the direction of Y. In the example shown by Fig. 3, comb-shaped first electrode (11) and second electrode (12) are arranged on the support (30) with facing each other and sandwiching the piezoelectric/electrostrictive element (20). In this case, a lower voltage can give a sufficient vibration since the vibration in the direction of Y is obtained utilizing a longitudinal effect of electric field-induced strain of the piezoelectric/electrostrictive element (20).

The piezoelectric/electrostrictive element (20) is a known piezoelectric/electrostrictive substance or an antiferroelectric substance, such as a ceramic material including lead zirconate, lead titanate, lead magnesium niobate, lead nickel niobate, lead zinc niobate, lead manganese niobate, lead antimony stannate, lead manganese tungstate, lead cobalt niobate, barium titanate, which may be employed alone or in combination. One especially preferred is a material whose major ingredient is a component consisting of lead zirconate, lead titanate and lead magnesium niobate. Such a material is preferred since it has high electromechanical binding coefficient and piezoelectric constant as well as a low reactivity with a support (30) upon sinterning the piezoelectric/electrostrictive element (20), whereby allowing a predetermined composition to be achieved constantly.

A ceramic material listed above supplemented with an oxide of lanthanum, calcium, strontium, molybdenum, tungsten, barium, niobium, zinc, nickel, manganese, cerium, cadmium, chromium, cobalt, antimony, iron, yttrium, tantalum, lithium, bismuth, tin and the like or a mixture thereof as well as other compounds may also be employed. For example, a ceramic material containing lead zirconate, lead titanate and lead magnesium niobate as major ingredients together with lanthanum and strontium is employed preferably, and it becomes more preferable when supplemented also with manganese because of an elevated mechanical quality coefficient (Q value) of the piezoelectric material, which gives an increase in Q value which is attributable to the material aspect in addition to the structural aspect of the reactive chip.

The first electrode (11) and the second electrode (12) are formed preferably from conductive metals which are solid at room temperature, such as a metal element of aluminum, titanium, chromium, iron, cobalt, nickel, copper, zinc, niobium, molybdenum, ruthenium, palladium, rhodium, silver, tin, tantalum, tungsten, iridium, platinum, gold, lead and the like as well as an alloy of any combination thereof. In addition, a cermet material obtained by dispersing the materials similar to those of the piezoelectric/electrostrictive element (20) or support (30) in any of the metals listed above may also be employed.

In order to form the vibration-generating part (40) and the vibration area (50) from the materials described above for example in the case of the structure shown in Fig. 1, the second electrode (12) is formed on the support (30) and then the piezoelectric/electrostrictive element (20) is sintered on this second electrode (12), and finally a first electrode (11) is formed. Alternatively, the first electrode (11), the second electrode (12) and the piezoelectric/electrostrictive element (20) are sintered altogether as being integrated on the support (30). The formation of the vibration area (50) by means of such an integrated sintering is preferred especially in the case of the structures shown in Fig. 2 and Fig. 3.

The first electrode (11), the second electrode (12) and their respective lead wires may be coated with an insulant at any sites which will be brought into contact with a target substance. Such an insulant may be an insulative glass or resin. The resin may for example be a fluorine resin having excellent chemical stability, such as ethylene tetrafluoride resin-based teflon (Teflon PTFE from DuPont), ethylene tetrafluoride/propylene hexafluoride copolymeric resin-based teflon (Teflon FEP), ethylene tetrafluoride/perfluoroalkyl vinyl ether copolymeric resin-based teflon (Teflon PFA), PTFE/PFA composite teflon and the like. A silicone resin (especially thermosetting silicone resin), epoxy resin and acryl resin may also be employed as appropriate for the purpose. It is also preferable to add an inorganic or organic filler to the insulative resin to adjust the rigidity of the vibration area (50).

Since a thinner vibration area (50) not only leads to a higher sensitivity upon measuring the resonance frequency described later but is associated with a problematically reduced rigidity, the entire thickness of the vibration area (50) consisting of the support (30) and the vibration-generating part (40) is preferably about 5 to 50 µm.

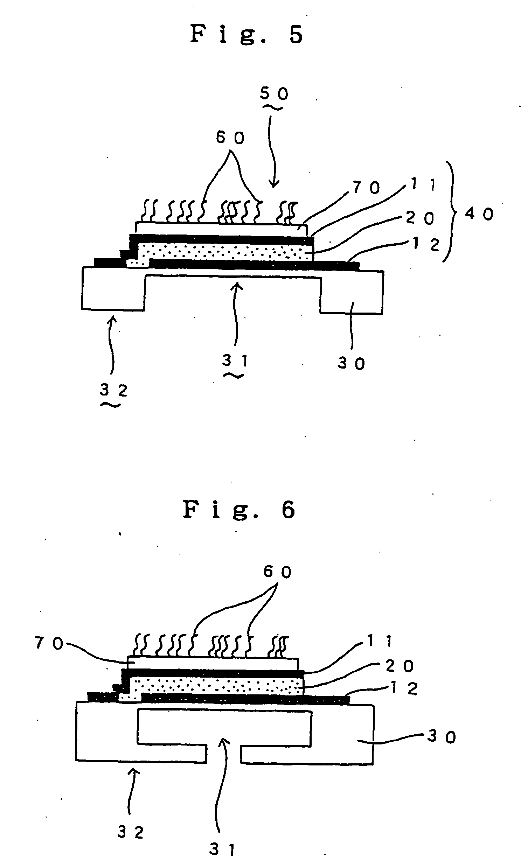

Fig. 4 shows a sectional view of one example of the reactive chip, of the invention. The reactive chip exemplified in this Fig. 4 has, on the surface of the support (30), the second electrode (12), the piezoelectric/electrostrictive element (20) and the first electrode (11) which are integrated altogether as a laminate. On the surface of the first electrode (11), capture probes (60) are provided. The capture probes (60) may be fixed directly on the surface of the vibration-generating part (40) as shown in Fig. 4, or may be fixed on the coating layer (70) which has been formed on the surface of the vibration-generating part (40) as shown in Fig. 5. Thus, this coating layer (70) consists of a material which facilitates the fixation of the capture probes (60) and may be selected appropriately based on the type of the capture probes (60) from a polynucleotide L lysin layer, a silane compound such as γ-aminopropyltriethoxysilane or its derivative, a biological adsorbent such as biotin/avidin, an organic polymer such as polyacrylamide or nylon membranes and the like.

In the examples shown in Fig. 4 and Fig. 5, the support (30) has the thin area (31) surrounded by the thick area (32) and the thin area (31) serves as the vibration area (50) having the vibration-generating part (40) formed thereon. By providing such a thin area (31) and a thick area (32), the rigidity of the reactive chip can be maintained and a preferable vibration can be generated in the vibration area (50). These thick area (32) and thin area (31) may be formed in such a structure in which the lower end of the thick area (32) is extended to form a cavity beneath the thin area (31) as shown in Fig. 6. Such a structure is preferable for the purpose of improving the overall rigidity of the support (30).

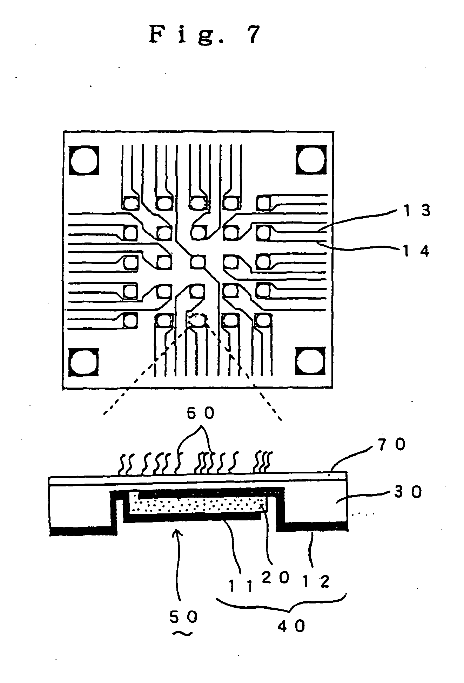

Moreover, an inventive reactive chip may have its vibration-generating part (40) as being provided below the thin area (31) of the support as shown in Fig. 7. Such a configuration allows a reactive chip having a flat surface to be obtained easily. Also because of freedom from any direct contact of the vibration-generating part (40) with a sample solution, the durability of the chip is improved, and the effect of noises upon measuring the resonance frequency described later can be eliminated, resulting in a further accurate measurement.

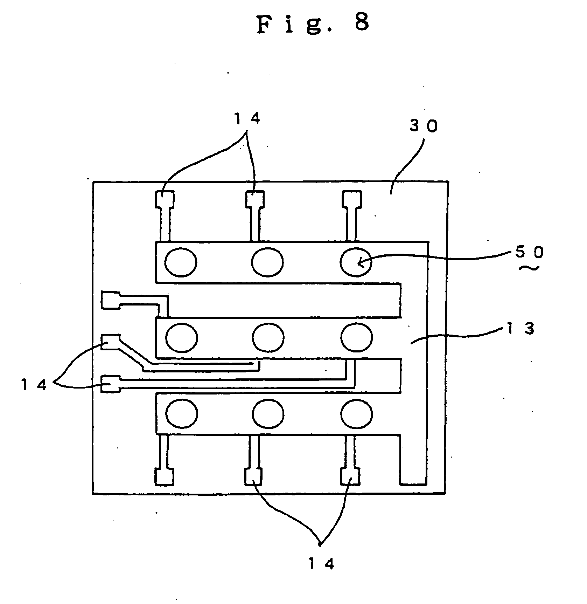

In an inventive reactive chip as still another embodiment, each lead wires (13) and (14) from the first electrode (11) and the second electrode (12) of respective vibration-generating parts (40) may be independent of those of any other vibration-generating part as shown in the plain view of Fig. 7. As a result, it becomes possible to vibrate each vibration area (50) at a different frequency or amplitude. Alternatively, it is also possible in a still another embodiment that any one of the lead wires of the first electrode (11) and the second electrode (12) is employed in common. For example, Fig. 8 shows the lead wire (13) from the first electrode (11) is employed in common, whereby simplifying a processing of lead wires. Also in the case where 4 or more vibration areas are arranged in a matrix, the electrode lead wires from the vibration areas in an identical line may be employed in common, whereby allowing the vibration areas in the identical line to vibrate at an identical amplitude.

The reactive chip has a means for measuring a resonance frequency of the vibration area. The principle and a typical methodology with regard to this measurement of resonance frequency are similar substantially to those disclosed in our previous application (JP-W-99/034176, JP-A-08-201265), and the measurement means can be established according to the method described in JP-W-99/034176 and JP-A-08-201265. Thus, a change in such a resonance frequency of the vibration area, typically upon adhesion of any exogenous substance to the vibration area or upon change in the specific gravity or viscosity of a sample solution in contact with the vibration area, can be detected as a change in an electric constant of a circuit containing the piezoelectric/electrostrictive element.

In a preferred reactive chip, the surface of the first electrode is a capture probe-fixing surface and the first electrode and the second electrode are connected not only with an alternating-current power source but also with a direct-current power source. Thus, this reactive chip can not only vibrate the vibration area upon supply of an alternating current to the first electrode and the second electrode but also apply a positive or negative charge to the first electrode as a capture probe-fixing surface. Such an application of an electrical charge can be conducted in accordance with the disclosure in JP-W-2001-501301.

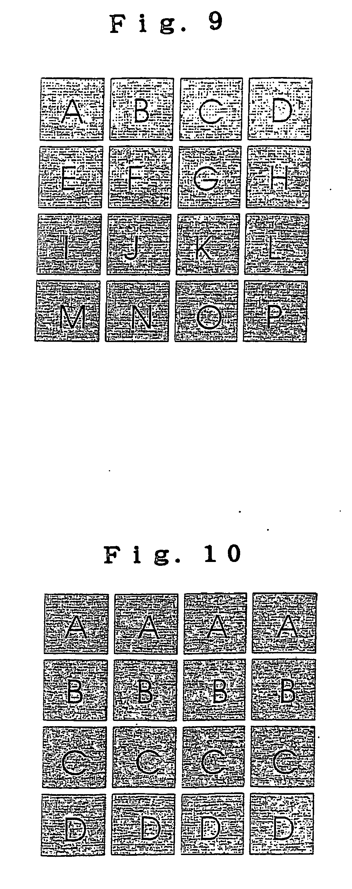

In the reactive chip, different capture probes may be fixed on different vibration areas. As used herein, the "different" means a difference in the base sequence of a DNA fragment or a difference in the amino acid sequence in a peptide. For example, different capture probes A to P are fixed on 16 vibration areas as shown in Fig. 9.

A method using the chip of Fig. 9 is a method for detecting a target substance which binds to a capture probe comprising bringing a labeled target substance-containing sample into contact with the capture probes on the reactive chip, while allowing the vibration area of the reactive chip to vibrate followed by terminating the vibration of the vibration area and detecting the target substance bound to the capture probe using the label as an index.

The method of this invention is characterized by an additional step for "vibrating a capture probe-fixing surface" upon detecting a target substance using an ordinary reactive chip. Thus, such a vibration allows the target substance in a sample solution in contact with the reactive chip to be diffused more extensively when compared with a spontaneous diffusion, resulting in a promotion of the specific binding between the target substance and the capture probe. In addition, by conducting a hybridization while vibrating the capture probes, any mismatch binding or non-specific adsorption can be eliminated or reduced. As a result, a DNA fragment whose single base is different (for example SNP) or a molecule whose tertiary structure is different can be identified as a binding with its respective corresponding capture probe.

The time period during which the vibration area is vibrated may be selected appropriately depending on the types of the capture probes or target substance, and may for example be 1 second to 32 hours in case of using as a DNA chip. The vibration frequency of the vibration area is about 10 to 1 MHz and the amplitude is about 0.001 to 10 µm.

In this method, the labeling of a target substance may be conducted using various materials depending on a sort of a target substance, such as an enzyme, radioisotope, fluorescent dye, fluorescent protein and the like. The enzyme may be any enzyme as long as it fulfills the relevant requirement such as a large turnover number, stability even upon binding to an antibody and an ability of staining a substrate specifically, and may for example be a peroxidase, β-galactosidase, alkaline phosphatase, glucose oxidase, acetylcholine esterase, glucose-6-phosphorylation dehydrogenase, malic aid dehydrogenase and the like. It is also possible to use an enzyme inhibitor or coenzyme. The substrate may be any known substance appropriate for the enzyme employed. For example 3,3',5,5'-tetramethylbenzidine can be employed when using a peroxidase as an enzyme, and p-nitrophenol can be employed when using an alkaline phosphatase as an enzyme. The radioisotope may for example be ¹²⁵I and ³H, and the fluorescent dye may for example fluorescence isothiocyanate (FITC) and tetramethylrhodamine isothiocyanate (TRITC). The fluorescent protein is a protein emitting a fluorescence when irradiated with an excitation light, such as a photogenic jellyfish-derived green fluorescent protein (GFP) or its variants EGFP, EYFP (yellow fluorescence), ECFP (blue fluorescence), DsRed1 (red fluorescence), DsRed2, as well as a Renilla-derived green fluorescent protein hrGFP and the like. The label and the target substance described above can be integrated for example via a hydrogen bond, hydrophobic bond, ionic bond, coordinate bond and the like. A fluorescent protein-labeled DNA fragment or fluorescent protein-labeled protein or peptide can be easily produced by a known gene engineering method since the polynucleotide sequence encoding the fluorescent protein is known.

In order to detect a target substance bound to a capture probe using the label mentioned above as an index, for example in the case of a enzyme label, a substrate capable of being decomposed by the enzymatic effect to develop a color is added, and the level of the decomposition of the substrate is measured optically to determine the enzymatic activity, which is converted into the level of the binding labeled substance and finally resulted in an amount of the target substance by caliculation comparison with standard. When using a radioisotope, the dose irradiated by the radioisotope is measured for example by a scintillation counter. When using a fluorescent dye or fluorescent protein, a device integrated with a fluorescent microscope can be used to measure a fluorescent intensity.

The reactive chip as one embodiment of the invention may have a "means for measuring a resonance frequency of the piezoelectric/electrostrictive element" as mentioned above. This reactive chip can be used in the method for detecting a target substance which binds to a capture probe comprising bringing a target substance-containing sample into contact with the capture probes on the reactive chip while allowing the vibration area of the reactive chip to vibrate, followed by detecting the target substance measuring the change in the resonance frequency of the piezoelectric/electrostrictive element as an index.

Thus, this method is a method for detecting a target substance using as an index a change in the resonance frequency of the vibration area generated as a result of the binding of the target substance to a capture probe. By this method, the detection can be accomplished without labeling a target substance. A continuous real-time measurement of the target substance binding level can also be conducted. It is also possible to label the target substance and to combine the detection using a label as an index, whereby accomplishing a more accurate detection.

This reactive chip can also be employed in the method for detecting a target substance, comprising bringing the sample into contact with the probe while allowing the vibration area of the reactive chip to vibrate and changing the temperature over a time period followed by detecting the target substance continuously measuring the change in the resonance frequency of the piezoelectric/electrostrictive element as an index. For example in the case of a DNA chip, the vibration area is vibrated while changing the hybridization reaction temperature to 38 to 98°C within a certain time period (for example at an interval of 10 minutes), during which the mass of the target DNA hybridized with the capture probe DNA is measured, whereby detecting Tm (melting point) of each labeled DNA.

A reactive chip of the invention may be provided wherein the surface of the first electrode is a capture probe-fixing surface and the first electrode and the second electrode are connected not only with an alternating-current power source but also with a direct-current power source. This reactive chip can be employed in the method for detecting a target substance which binds to a capture probe comprising bringing a labeled target substance-containing sample into contact with the probe on the reactive chip while allowing the vibration area of the reactive chip to vibrate followed by terminating the vibration of the vibration area, followed by applying a negative charge to the first electrode as a capture probe-fixing surface for a certain time period, followed by detecting the target substance bound to the capture probe using the label as an index. This method may be combined with a method using a nano-chip disclosed for example in JP-W-09-503307 and JP-W-2001-501301, especially for the purpose of using the reactive chip as a DNA chip. By combining the vibration of the capture probe-fixing surface and the negative charge on the capture probe, the mismatch binding between labeled DNA and probe DNA can efficiently be eliminated.

The direct current may be selected appropriately based on the solution employed, size of the vibration area, DNA concentration and the like, and may be 0.1 to 1000 nA, preferably 1 to 30 nA, which is applied for a period of 1 to 180 seconds, preferably 10 to 60 seconds.

The reactive chip may employ an arrangement of three or more vibration areas in a line or four or more vibration areas in a matrix of n × m wherein n is 2 or more and m is 2 or more, with identical capture probes being fixed in each vibration area in identical lines.

As used herein, the "identical" means a complete identity in the base sequence of a DNA fragment or a complete identity in the amino acid sequence in a peptide. For example as shown in Fig. 10, identical capture probes (A) are fixed on 4 vibration areas on the first line, while each identical capture probes (B), (C) and (D) are fixed on the 2nd to 4th lines, respectively. This reactive chip can be used in the method for detecting the affinity of each of different target substances to a capture probe comprising bringing different labeled target substances into contact with the capture probes on a reactive chip while allowing each vibration area of the vibration surfaces of the reactive chip arranged in an identical line to vibrate at different amplitudes followed by terminating the vibration of the vibration areas and detecting a degree of the affinity of each target substance binding to each respective capture probe toward the probe using the label as an index.

Thus, according to this method, the hybridization of the target substance with the capture probe is conducted while vibrating each vibration area arranged in an identical line at different amplitudes, and then an amount of the target substance bound to the probe is detected using the label as an index, whereby enabling the sorting of the target substance in the order of the higher affinity. In another method of the invention employing the reactive chip having means for measuring the resonance frequency, a continuous sorting can be accomplished using a mass as an index, which measured on the basis of the difference in the resonance frequency. Another method of the invention enables hybridization while fluctuating the temperature. Also in a method of the invention employing the reactive chip of claim 5, the affinity between the labeled DNA and the probe DNA can be detected at a further higher accuracy by combining the vibration of the capture probe-fixing surface and the negative charge on the capture probe.

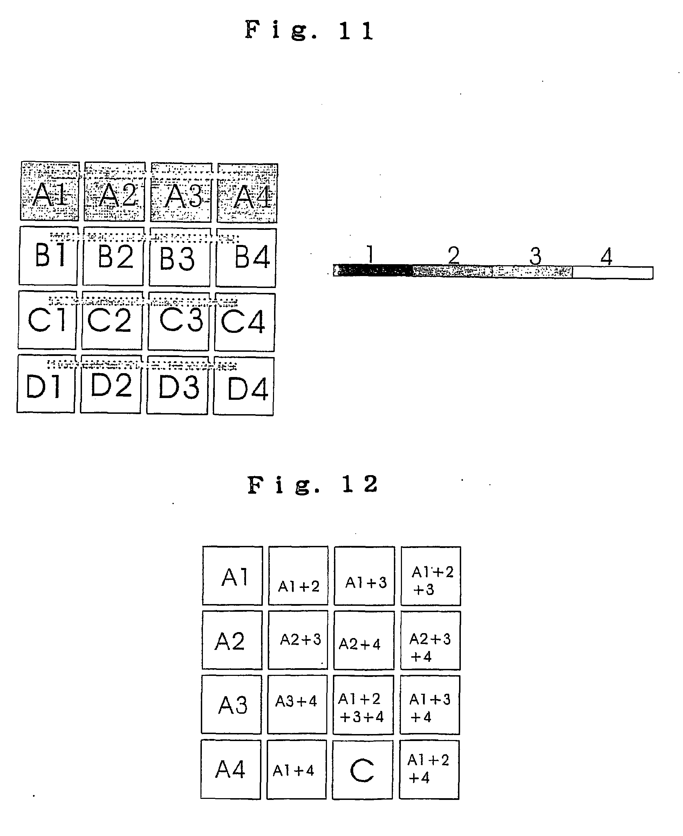

The reactive chip of the invention may employ an arrangement of three or more vibration areas in a line or four or more vibration areas in a matrix of n × m wherein n is 2 or more and m is 2 or more, with a capture probe which binds to a different site of a target substance being fixed in each vibration area in an identical line. As used herein, "binding to a different site of a target substance" means, for example, binding to the region of a different base sequence of a DNA fragment or binding to the region of a different full-length amino acid sequence of a peptide. For example, the capture probes to A4 corresponding to the 4 regions sequentially from the end of a chromosome DNA (A) are fixed on the four vibration areas in the first line, and the capture probes corresponding to the four regions of the chromosomes (B) to (D) are fixed on the 2nd to 4th lines, respectively, as shown in Fig. 11. Also as shown in Fig. 12, the capture probes corresponding to various combinations of the four regions sequentially from the end of the chromosome DNA (A) are fixed on the vibration areas arranged in a 4 x 4 matrix.

This reactive chip can be employed in the method for detecting a mutation in a target substance comprising bringing a labeled target substance-containing sample into contact with capture probes on the reactive chip of the 16th invention while allowing the vibration areas of the reactive chip arranged in an identical line to vibrate, followed by terminating the vibration of the vibration area, followed by detecting the target substance bound to the capture probe using the label as an index. As used herein, a "mutation" means a deletion, substitution, insertion, amplification, repeat and the like. More typically, it means such a mutation in a chromosomal DNA or a mutation in an mRNA or cDNA corresponding thereto as well as a similar mutation in a protein or peptide as an expression product thereof.

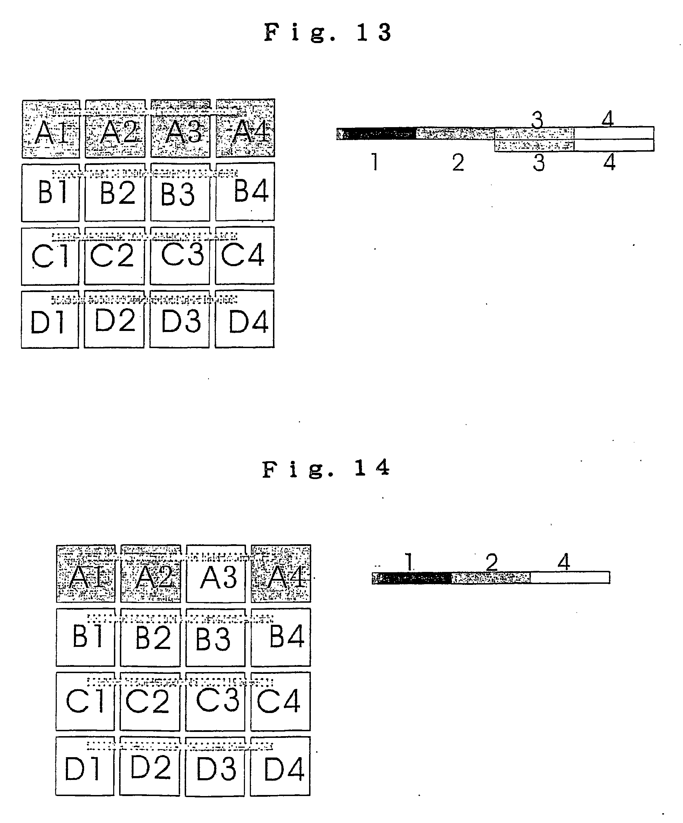

For example, when the capture probes are fixed in the configration shown in Fig. 11, the target substance is bound individually to each probe as shown in the right of the Fig. 11 in case of normal the target substance (chromosomal DNA). However, when the level of the binding to the probes 3 and 4 is higher by two times as shown in Fig. 13, then it may be detected that the amplification of the chromosomal DNA regions 3-4 corresponding to these probes 3 and 4 occured. Also as shown in Fig. 14, when the binding occurs to the probes 1, 2 and 4 but does not to the probe 3, then the deletion of the chromosomal DNA region 3 corresponding to the probe 3 can be detected.

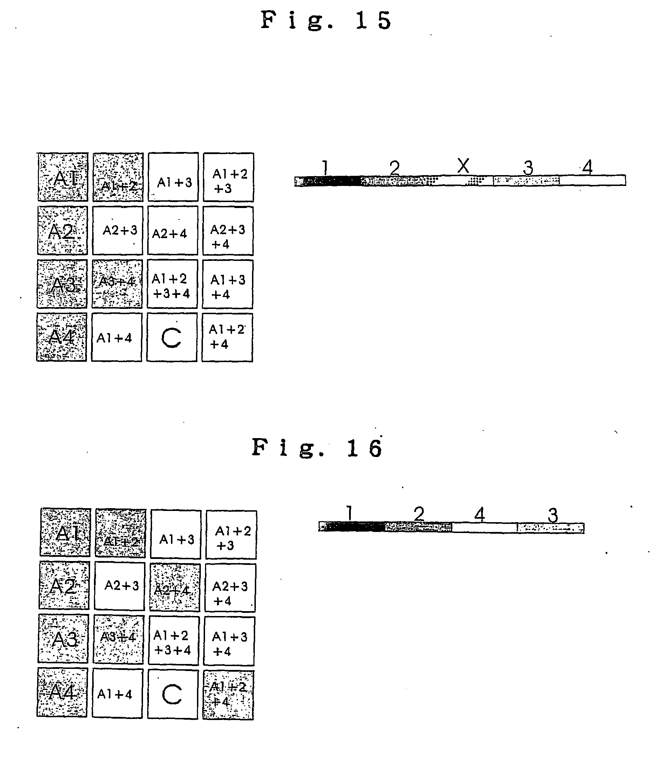

Also for example, by means of fixing the capture probes in the configration shown in Fig. 12, it becomes possible to detect an insertion or substitution of a chromosomal DNA. For example, as shown in the left column of Fig. 15, when a target substance (chromosomal DNA) binds to the probes of A1 to A4 alone and also to the probes of A1+2 and A3+4, then an insertion of X sequence between the chromosomal DNA regions 2 and 3 can be detected. Also as shown in the left column of Fig. 16, when the chromosomal DNA binds to the probes of A1 to A4 alone and also to the probes of A1+2, A2+4, A3+4 and A1+2+4, then a substitution of the chromosomal DNA region 3 with 4 can be detected.

The method of the invention using the reactive chip having the means for measuring the resonance frequency enables the detection of a mutation in a target substance using as an index a mass measured on the basis of the difference in the resonance frequency. For example, the mass increased by 6/4 times in Fig. 13 and the mass reduced by 3/4 times in Fig. 14 are detected. A method employing the reactive chip of claim 5 enables a further highly accurate detection of a mutation in a labeled DNA by combining means of a vibration of the capture probe-fixing surface and a negative charge on the capture probe.

It is a matter of course that the invention disclosed here is not limited by the examples described above, and various modifications can be made in the detail of the invention.

### Industrial Applicability

As detailed above, the invention of this application provides a novel chip capable of reducing a reaction period, applying a wide-ranging target substance, preventing a mismatch binding efficiently, and enabling a highly accurate detection is provided. A novel method for detecting a target substance employing this reactive chip is also provided. In this detection method, the detection even of a slight difference in the target substance, which has been impossible when using a conventional reactive chip, becomes possible.

## Claims

1. A reactive chip comprising capture probes (60) fixed on each of three or more vibration areas (50) arranged spaced apart on a support (30), the capture probes (60) being able to bind to a target substance,
wherein each vibration area (50) has a vibration-generating part (40) having a first electrode (11) and a second electrode (12) between which a piezoelectric/electrostrictive element (20) is sandwiched,
wherein at each said vibration area (50) the support has a thin area (31) surrounded by a thick area (32) and has the vibration-generating part (40) on a surface of the thin area (31), whereby said thin area is vibrated by operation of said vibration-generating part, and
**characterized in that** at each said vibration area (50) said capture probes (60) are attached on said thin area (31) so as to move with said thin area when said thin area (31) is vibrated by said vibration-generating part (40).

2. The reactive chip of claim 1, wherein said capture probes are attached directly to said first electrode of said vibration-generating part or to a layer (70) on said vibration-generating part (70) or are attached to a layer (70) on one surface of said thin area (31) when said vibration-generating part is on the other surface of said thin area (31).

3. The reactive chip of claim 1 or 2, wherein for each said vibration-generating part a lead wire for each of the first and second electrodes is independent from the respective lead wire of the electrodes of each other said vibration-generating part.

4. The reactive chip of claim 1 or 2, wherein a lead wire for one of the first and second electrodes is common for said vibration-generating parts.

5. The reactive chip of claim 1, wherein the surface of the first electrode is a capture probe-fixing surface and the first electrode and the second electrode are connected not only with an alternating-current power source but also with a direct-current power source.

6. The reactive chip of any one of claims 1 to 5. wherein the kind of capture probes fixed on one vibration area (50) is different from the capture probes of other vibration areas (50).

7. The reactive chip of any one of claims 1 to 6. which employs an arrangement of three or more vibration areas (50) in a line or four or more vibration areas (50) in a matrix of n × m wherein n is 2 or more and m is 2 or more, with identical capture probes being fixed in each vibration area in identical lines.

8. The reactive chip of any one of claims 1 to 6, which employs an arrangement of three or more vibration areas (50) in a line or four or more vibration areas in a matrix of n × m wherein n is 2 or more and m is 2 or more, with a capture probe which binds to a different site of a target substance being fixed in each vibration area (50) in an identical line.

9. Method of detecting a target substance, using a reactive chip according to any one of claims 1 to 8.

10. Method according to claim 9, which comprises bringing a sample containing a labelled target substance into contact with the capture probes on the reactive chip while vibrating said vibration area (50) to vibrate said capture probes, followed by terminating the vibration of the vibration area (50) and detecting the target substance bound to the capture probes using the label as an index.

## Patentansprüche

1. Reaktiver Chip, der Folgendes umfasst: Auffangsonden (60), die in jedem von drei oder mehreren Schwingungsbereichen (50) befestigt sind, die voneinander beabstandet auf einem Träger (30) angeordnet sind, wobei die Auffangsonden (60) in der Lage sind, an eine Zielsubstanz zu binden,
wobei jeder Schwingungsbereich (50) einen Schwingungen erzeugenden Teil (40) mit einer ersten Elektrode (11) und einer zweiten Elektrode (12) aufweist, zwischen denen ein piezoelektrisches/elektrostriktives Element (20) angeordnet ist,
wobei der Träger in jedem Schwingungsbereich (50) einen dünnen Bereich (31) aufweist, der von einem dicken Bereich (32) umgeben ist, und den Schwingungen erzeugenden Teil (40) auf einer Oberfläche des dünnen Bereichs (31) aufweist, wobei der dünne Bereich durch die Betätigung des Schwingungen erzeugenden Teils in Schwingung versetzt wird, und
**dadurch gekennzeichnet, dass** die Auffangsonden (60) in jedem Schwingungsbereich (50) auf dem dünnen Bereich (31) befestigt sind, um sich mit dem dünnen Bereich zu bewegen, wenn der dünne Bereich (31) durch den Schwingungen erzeugenden Teil (40) in Schwingungen versetzt wird.

2. Reaktiver Chip nach Anspruch 1, worin die Auffangsonden direkt an der ersten Elektrode des Schwingungen erzeugenden Teils oder an einer Schicht (70) auf dem Schwingungen erzeugenden Teil (70) befestigt sind oder an einer Schicht (70) auf einer Oberfläche des dünnen Bereichs (31) befestigt sind, wenn sich der Schwingungen erzeugende Teil auf der anderen Oberfläche des dünnen Bereichs (31) befindet.

3. Reaktiver Chip nach Anspruch 1 oder 2, worin ein Anschlussdraht der ersten und der zweiten Elektrode jedes der Schwingungen erzeugenden Teile von dem entsprechenden Anschlussdraht der Elektroden jedes anderen Schwingungen erzeugenden Teils unabhängig ist.

4. Reaktiver Chip nach Anspruch 1 oder 2, worin die Schwingungen erzeugende Teile gemeinsam einen Anschlussdraht für eine Elektrode der ersten und zweiten Elektrode haben.

5. Reaktiver Chip nach Anspruch 1, worin die Oberfläche der ersten Elektrode eine Oberfläche zur Befestigung von Auffangproben ist und die erste Elektrode und die zweite Elektrode nicht nur mit einer Wechselstromquelle, sondern auch mit einer Gleichstromquelle verbunden sind.

6. Reaktiver Chip nach einem der Ansprüche 1 bis 5, worin sich die Art der Auffangsonden die auf einem Schwingungsbereich (50) befestigt ist, von den anderen Auffangsonden der anderen Schwingungsbereiche (50) unterscheidet.

7. Reaktiver Chip nach einem der Ansprüche 1 bis 6, bei dem eine Anordnung von drei oder mehr Schwingungsbreichen (50) in einer Zeile oder vier oder mehr Schwingungsbereichen (50) in einer Matrix von n x m eingesetzt wird, worin n ≥ 2 ist und m ≥ 2 ist, wobei in jedem Schwingungsbereich in identischen Zeilen identische Auffangsonden befestigt sind.

8. Reaktiver Chip nach einem der Ansprüche 1 bis 6, bei dem eine Anordnung von drei oder mehr Schwingungsbereichen (50) in einer Zeile oder vier oder mehr Schwingungsbereichen in einer Matrix von n x m eingesetzt wird, worin n ≥ 2 ist und m ≥ 2 ist, wobei in jedem Schwingungsbereich (50) in einer identischen Zeile eine Auffangsonde befestigt ist, wobei diese jeweils an verschiedene Stellen einer Zielsubstanz bindet.

9. Verfahren zur Detektion einer Zielsubstanz unter Verwendung eines reaktiven Chips nach einem der Ansprüche 1 bis 8.

10. Verfahren nach Anspruch 9, das Folgendes umfasst: In-Kontakt-Bringen einer Probe, die eine markierte Zielsubstanz enthält, mit den Auffangsonden auf dem reaktiven Chip, während der Schwingungsbereich (50) in Schwingungen versetzt wird, um die Auffangsonden in Schwingungen zu versetzen, anschließend das Beenden des Schwingens des Schwingungsbereichs (50) und das Detektieren der an die Auffangsonden gebundenen Zielsubstanz unter Verwendung der Markierung als Index.

## Revendications

1. Puce réactive comprenant des sondes de capture (60) fixées sur chacune de trois ou plusieurs zones de vibration (50) agencées d'une manière espacée sur un support (30), les sondes de capture (60) étant aptes à se lier à une substance cible,
où chaque zone de vibration (50) possède une partie de génération de vibrations (40) ayant une première électrode (11) et une deuxième électrode (12) entre lesquelles un élément piézoélectrique/électrostrictif (20) est pris en sandwich,
où à chaque zone de vibration précitée (50), le support possède une zone mince (31) entourée d'une zone épaisse (32) et possède une partie de génération de vibrations (40) sur une surface de la zone mince (31), par quoi ladite zone mince est amenée à vibrer par le fonctionnement de ladite partie de génération de vibrations, et
**caractérisée en ce que**, à chaque zone de vibration précitée (50), lesdites sondes de capture (60) sont fixées sur ladite zone mince (31) de manière à se déplacer avec ladite zone mince lorsque ladite zone mince (31) est amenée à vibrer par ladite partie de génération de vibrations (40).

2. Puce réactive selon la revendication 1, dans laquelle lesdites sondes de capture sont attachées directement à ladite première électrode de ladite partie de génération de vibrations ou à une couche (70) sur ladite partie de génération de vibrations (70) ou sont attachées à une couche (70) sur une surface de ladite zone mince (31) lorsque ladite partie de génération de vibrations se trouve sur l'autre surface de ladite zone mince (31).

3. Puce réactive selon la revendication 1 ou 2, dans laquelle pour chaque partie de génération de vibrations, un fil conducteur pour chacune des première et deuxième électrodes est indépendant du fil conducteur respectif des électrodes de chaque autre de ladite partie de génération de vibrations.

4. Puce réactive selon la revendication 1 ou 2, dans laquelle un fil conducteur pour une des première et deuxième électrodes est commun auxdites parties de génération de vibrations.

5. Puce réactive selon la revendication 1, dans laquelle la surface de la première électrode est une surface de fixation de sonde de capture, et la première électrode et la deuxième électrode sont connectées non seulement à une source de courant alternatif mais également à une source de courant direct.

6. Puce réactive selon l'une quelconque des revendications 1 à 5, dans laquelle le type de sonde de capture fixé sur une zone de vibration (50) diffère des sondes de capture des autres zones de vibration (50).

7. Puce réactive selon l'une quelconque des revendications 1 à 6, qui utilise un agencement de trois ou plusieurs zones de vibrations (50) en une ligne ou bien quatre ou plus de zones de vibration (50) en une matrice de n × m où n est 2 ou plus et m est 2 ou plus, des sondes de capture identiques étant fixées dans chaque zone de vibration dans des lignes identiques.

8. Puce réactive selon l'une quelconque des revendications 1 à 6, qui utilise un agencement de trois ou plusieurs zones de vibration (50) en une ligne ou bien quatre ou plusieurs zones de vibration dans une matrice de n × m où n est 2 ou plus et m est 2 ou plus, une sonde de capture qui se lie à un site différent d'une substance cible étant fixée dans chaque zone de vibration (50) dans une ligne identique.

9. Procédé de détection d'une substance cible, en utilisant une puce réactive selon l'une quelconque des revendications 1 à 8.

10. Procédé selon la revendication 9, qui comprend l'amenée d'un échantillon contenant une substance cible marquée en contact avec les sondes de capture sur la puce réactive tout en faisant vibrer ladite zone de vibration (50) pour faire vibrer lesdites sondes de capture, suivie de la terminaison de la vibration de la zone de vibration (50) et de la détection de la substance cible liée aux sondes de capture en utilisant l'indicateur comme indice.
